# EUROPÄISCHE PATENTSCHRIFT

(11) **EP 1 370 600 B1**
(45) Veröffentlichungstag und Bekanntmachung des Hinweises auf die Patenterteilung: **13.10.2004**
(21) Anmeldenummer: 01991752.5
(22) Anmeldetag: 22.11.2001
(51) Int. Cl.: C08G 65/30, C07C 41/03, B01J 27/26, C08G 65/12

(54) **VERFAHREN ZUR AUFARBEITUNG VON POLYETHERALKOHOLEN**
METHOD FOR PROCESSING POLYETHER ALCOHOLS
PROCEDE DE TRAITEMENT D'ALCOOLS DE POLYETHER

(30) Priorität: 22.11.2000 DE 10057891; 22.11.2000 DE 10057892
(43) Veröffentlichungstag der Anmeldung: 17.12.2003
(73) Patentinhaber: BASF AKTIENGESELLSCHAFT, 67056 Ludwigshafen (DE)
(72) Erfinder: BARTH, Thomas DI, deceased (DE); HEIDER, Wolfgang, 67434 Neustadt (DE); BOHRES, Edward Dr., 68161 Mannheim (DE); GROSCH, Georg, Heinrich, 67098 Bad Dürkheim (DE); VOSS, Hartwig, 67227 Frankenthal (DE); HÖPPNER, Gerd, 01987 Schwarzheide (DE); HARRE, Kathrin, 01109 Dresden (DE); PAREDIS, Els, B-2520 Ranst (BE); WINKLER, Jürgen, 01987 Schwarzheide (DE); STÖSSER, Michael, 67141 Neuhofen (DE); SAGER, Wilfried, 67112 Mutterstadt (DE)
(74) Vertreter: Isenbruck, Günter, Dr.
(86) Internationale Anmeldenummer: PCT/EP2001/013619
(87) Internationale Veröffentlichungsnummer: WO 2002/042356

(56) Entgegenhaltungen:
- EP-A- 0 400 554
- EP-A- 0 654 302
- EP-A- 0 665 254
- WO-A-94/29362
- WO-A-98/44022
- DE-A- 4 115 149
- US-A- 4 306 943
- US-A- 5 689 012
- US-A- 6 013 596

## Beschreibung

Die vorliegende Erfindung betrifft ein Verfahren zur Aufarbeitung von Polyetheralkoholen (Polyetherole), die durch ring-öffnende Polymerisation von Epoxiden mit OH-funktionellen Startern unter Verwendung eines heterogenen Katalysators hergestellt wurden, wobei der heterogene Katalysator durch ein Membrantrennverfahren vom Polyetherol abgetrennt wird, sowie ein Verfahren zur Herstellung derartiger Polyetherole, das einen entsprechenden Aufarbeitungsschritt umfasst. Darüber hinaus betrifft die vorliegende Erfindung die Verwendung der so erhaltenen Polyetherole in der Automobilindustrie in Treibstoffzusammensetzungen, als Treibstoffadditiv, in Bremsflüssigkeiten, in Polyurethanen, insbesondere Weichschaumstoffen, sowie als Tenside oder Lösemittel.

Die Herstellung von Polyetheralkoholen sowie deren Aufarbeitung sind bekannt.

So besteht ein vielfach verwendetes Verfahren zur Herstellung von Polyetherolen darin, die ring-öffnende Polymerisation in Gegenwart löslicher basischer Katalysatoren, wie z.B. Natrium-, Kalium- oder Cäsiumhydroxid, durchzuführen. Diese homogen-basischen Katalysatoren müssen nach der Polyetherol-Herstellung in der Regel wieder aus selbigem entfernt werden, da sie bei deren weiterer Verwendung stören. Zur Entfernung derartiger löslicher basischer Katalysatoren werden im Stand der Technik beispielsweise die Fällung der Alkalimetallionen als Phosphate, Chloride oder Carbonate mit anschließender Abtrennung des Alkalisalzes, sowie die Behandlung des erhaltenen Produktgemischs mit anorganischen oder organischen Kationenaustauschem beschrieben. Hierzu verweisen wir auf die US 4 306 943, EP-A 0 102 508 (Phosphate) sowie die DE-A 43 36 923 (Carbonate).

Neben löslichen basischen Katalysatoren werden im Stand der Technik auch unlösliche basische Katalysatoren, wie Magnesiumhydroxid oder Hydrotalcit, für die ring-öffnende Polymerisation von Epoxiden zur Herstellung von Polyethermonoolen und Polyetherpolyolen verwendet. Diese Katalysatoren werden in der Regel nach der Synthese ebenfalls vom erhaltenen Polyetherol abgetrennt, i. d. R. Tiefenfiltration, wie in der DE-A 41 15 149 bzw. der DE-A 40 34 305 beschrieben.

Ein weiterer Weg zur Herstellung von Polyetherolen durch ring-öffnende Polymerisation von Epoxiden ist die Verwendung von Multimetallcyanid-Katalysatoren, auch als DMC-Katalysatoren bezeichnet, wobei hier vorzugsweise Zinkhexacyanometallate verwendet werden. Diesbezüglich verweisen wir auf die DE-A 199 57 105.8, die DE-A 198 40 846.3, sowie die WO 98/44022 und den jeweils darin zitierten Stand der Technik.

Darüber hinaus werden im Stand der Technik mehrere Methoden zur Abtrennung der Multimetallcyanid-Katalysatoren aus dem Polyetherol beschrieben, wobei wir diesbezüglich nochmals auf die DE-A 199 57 105.8 und den darin ausführlich referierten Stand der Technik Bezug nehmen.

Dabei betrifft die DE-A 199 57 105.8 ein Verfahren zur Aufarbeitung von Polyetheralkoholen, in dem die als Katalysator verwendete Multimetallcyanidverbindung nach der ring-öffnenden Polymerisation durch Sedimentation aus dem Polyetheralkohol entfernt wird. Gemäß diesem Verfahren ist es möglich, den Katalysator chemisch unverändert aus dem Polyetheralkohol zurückzuerhalten und diesen dann in einer erneuten Herstellung von Polyetheralkoholen einzusetzen. Demgegenüber betreffen die in der DE-A 199 57 105.8 als Stand der Technik zitierten Druckschriften jeweils Verfahren, in denen der verwendete DMC-Katalysator in einer Form anfällt, in der er nicht wiederverwendet werden kann oder aber der DMC-Katalysator zerstört wird.

Somit lag der vorliegenden Erfindung die Aufgabe zugrunde, ein weiteres Verfahren zur Aufarbeitung von Polyetherolen bereitzustellen, das es ermöglichen sollte, den darin verwendeten heterogenen Katalysator ohne großen, insbesondere apparativen, Aufwand in einer Form abzutrennen, in der dieser Katalysator erneut zur Herstellung von Polyetherolen verwendet werden kann. Dies ist insbesondere im Hinblick auf die erfindungsgemäss verwendeten DMC-Katalysatoren von großer wirtschaftlicher Bedeutung, da diese Katalysatoren in ihrer Herstellung sehr teuer sind. Diese und weitere Aufgaben werden durch das erfindungsgemäße Verfahren gelöst

Demgemäss betrifft die vorliegende Erfindung ein Verfahren zur Aufarbeitung von Polyetherolen, die durch ring-öffnende Polymerisation von Epoxiden mit OH-funktionellen Startern unter Verwendung mindestens eines heterogenen Katalysators hergestellt wurden, dadurch gekennzeichnet, dass der mindestens eine heterogene Katalysator durch ein Membrantrennverfahren vom Polyetherol abgetrennt wird, wobei der heterogene Katalysator ein Multimetallcyanid-Katalysator ist.

Im Rahmen des erfindungsgemäßen Verfahrens kann der heterogene Katalysator nach der Abtrennung vom Polyetherol von selbigem befreit werden. Dies kann durch Waschen, beispielsweise mit Wasser oder einem organischen Lösungsmittel, erfolgen. Danach kann er in eine Form überführt werden, z.B. durch Trocknung, in der er zur Herstellung von Polyetherolen eingesetzt werden kann. Dies kann beispielsweise durch Dispergieren in Lösungsmitteln erfolgen. Diese Variante wird insbesondere dann durchgeführt, wenn der Katalysator nach der Abtrennung zur Herstellung eines anderen Polyetherols eingesetzt werden soll, und Verunreinigungen bei der Herstellung des anderen Polyetherols durch noch anhaftende Reste des ersten Polyetherols vermieden werden sollen.

Es ist jedoch auch möglich, den heterogenen Katalysator ohne weitere Aufarbeitung direkt wieder zur Herstellung eines Polyetherols einzusetzen. Diese Verfahrensvariante wird insbesondere dann angewendet, wenn der Katalysator nach der Aufarbeitung zur Herstellung des gleichen Polyetherols eingesetzt werden soll bzw. wenn geringfügige Verunreinigungen bei der Herstellung eines anderen Polyetherols durch das erste Polyetherol nicht von Bedeutung sind.

Vorzugsweise wird das erfindungsgemäße Verfahren so durchgeführt, daß der heterogene Katalysator während der Abtrennung vom Polyetherol aufkonzentriert wird und als konzentrierte Suspension im Polyetherol gewonnen wird. Wie oben erwähnt, kann diese konzentrierte Katalysatorsuspension anschließend wieder zur Synthese eines Polyetherols eingesetzt werden.

Das erfindungsgemäße Aufarbeitungsverfahren kann kontinuierlich oder diskontinuierlich durchgeführt werden, d.h. das nach der ring-öffnenden Polymerisation erhaltene Produktgemisch kann entweder kontinuierlich oder diskontinuierlich durch die Trennvorrichtung hindurchgeführt werden, wobei, wie oben erwähnt, vorzugsweise der Katalysator dabei aufkonzentriert wird.

Dabei wird der Teil des Polyetherols, der durch die Trennvorrichtung hindurchgeht, und damit im wesentlichen frei ist vom heterogenen Katalysator, als "Permeat" bezeichnet, während der verbleibende Anteil des Polyetherols, in dem sich vorzugsweise der Feststoff aufkonzentriert, als "Retentat" bezeichnet wird.

Als heterogene Katalysatoren werden Katalysatoren aus der Klasse der Multimetallcyanid-Verbindungen (DMC-Katalysatoren) im erfindungsgemäßen Herstell- bzw. Aufarbeitungsverfahren eingesetzt bzw. abgetrennt.

DMC-Katalysatoren werden eingesetzt, da sie deutlich aktiver bezüglich der Polymerisation von Epoxiden sind, als alle anderen bekannten Klassen an heterogenen Katalysatoren und deshalb in sehr viel geringeren Konzentrationen eingesetzt werden können. Das hat insbesondere im Zusammenhang mit dem erfindungsgemäßen Aufarbeitungsverfahren den Vorteil, dass man bei dem gleichen Katalysatorgehalt am Endpunkt der Aufkonzentration durch das Membrantrennverfahren eine höhere Aufkonzentrierungsrate und eine höhere Ausbeute an Polyetherol bzw. bei Rückführung einer den abgetrennten Katalysator umfassenden Suspension in die Herstellung des Polyetherols die Rückführrate des vorher als Produkt erhaltenen Polyetherols in diese (erneute) Herstellung des Polyetherols geringer ist.

Ferner zeigen DMC-Katalysatoren ein Phänomen, das in der Literatur mit dem Schlagwort "differenzielle Katalyse" bezeichnet wird. Unter differenzieller Katalyse wird verstanden, daß bei der Alkoxylierung eines Gemisches von Startern verschiedener Molmassen die Starter mit den niedrigsten Molmassen bevorzugt alkoxyliert werden, so dass es im Laufe der Polymerisationsreaktion zu einer Nivellierung der Molmassenunterschiede kommt. Beim erfindungsgemäßen Aufarbeitungsverfahren erweist sich gerade diese Eigenschaft als besonders günstig, da der abgetrennte Katalysator, sofern er erneut eingesetzt wird, bereits als Suspension im Endprodukt, das heißt, dem fertigen Polyetherol, mit hoher Molmasse eingesetzt wird. Durch die differenzielle Katalyse wird vermieden, dass sich durch die Rückführung des Katalysators in Suspension mit bereits fertigem Polyetherol in die Herstellung von neuem Polyetherol die Qualität des herzustellenden neuen Polyetherols verschlechtert.

Bezüglich der im Rahmen des erfindungsgemäßen Herstell- bzw. Aufarbeitungsverfahrens einsetzbaren bzw. abzutrennenden DMC-Katalysatoren existieren keinerlei Beschränkungen. Bezüglich dieser Katalysatoren und deren Herstellung wird wiederum auf die DE-A 199 57 105.8, die DE-A 198 40 846.3, sowie die WO 98/44022 verwiesen.

Insbesondere bevorzugt werden folgende DMC-Katalysatoren eingesetzt: Doppelmetallcyanid-Katalysatoren, wie sie in der DE-A 198 40 846.3 beschrieben sind, enthaltend Doppelmetallcyanid-Katalysatoren, enthaltend eine Doppelcyanidkomplexverbindung der allgemeinen Formel (I)

M¹ ₐ[M²(CN)_{b}(A)_{c}]_{d} ^{·} fM¹ _{g}Xₙ ^{·} h(H₂O) · eL^{·}kP, (I)

in der
- M¹: mindestens ein Metallion, ausgewählt aus der Gruppe bestehend aus Zn²⁺, Fe²⁺, Co³⁺, Ni²⁺, Mn²⁺, Co²⁺, Sn²⁺, Pb²⁺, Mo⁴⁺, Mo⁶⁺, Al³⁺, V⁴⁺, V⁵⁺, Sr²⁺, W⁴⁺, W⁶⁺, Cr²⁺, Cr³⁺, Cd²⁺,
- M²: mindestens ein Metallion, ausgewählt aus der Gruppe bestehend aus Fe²⁺, Fe³⁺, Co²⁺, Co³⁺, Mn²⁺, Mn³⁺, V⁴⁺, V⁵⁺, Cr²⁺, Cr³⁺, Rh³⁺, Ru²⁺, Ir³⁺,
wobei M¹ und M² verschieden sind,
- A: ein Anion, ausgewählt aus der Gruppe, bestehend aus Halogenid, Hydroxyd, Sulfat, Carbonat, Cyanid, Thiocyanat, Isocyanat, Cyanat, Carboxylat, Oxalat oder Nitrat,
- X: ein Anion, ausgewählt aus der Gruppe, bestehend aus Halogenid, Hydroxyd, Sulfat, Carbonat, Cyanid, Thiocyanat, Isocyanat, Cyanat, Carboxylat, Oxalat oder Nitrat,
- L: einen mit Wasser mischbaren Liganden, ausgewählt aus der Gruppe, bestehend aus Alkoholen, Aldehyden, Ketonen, Ethern, Polyethern, Estern, Harnstoffen, Amiden, Nitrilen und Sulfiden,
- k: eine gebrochene oder ganze Zahl größer oder gleich Null ist, und
- P: ein organischer Zusatzstoff ist,
wobei a, b, c, d, g und n so ausgewählt sind, dass die Elektroneutralität der Verbindung gewährleistet ist,
- e: die Koordinationszahl des Liganden größer 0 oder 0
- f: eine gebrochene oder ganze Zahl größer 0 oder 0 und
- h: eine gebrochene oder ganze Zahl größer 0 oder 0
bedeuten.

Als organische Zusatzstoffe sind zu nennen:

Polyether, Polyester, Polycarbonate, Polyalkylenglykolsorbitanester, Polyakylenglykolglycidylether, Polyacrylamid, Poly(acrylamid-co-acrylsäure), Polyacrylsäure, Poly(acrylamid-co-maleinsäure), Polyacrylnitril, Polyalkylacrylate, Polyalkylmethacrylate, Polyvinylmethylether, Polyvinylethylether, Polyvinylacetat, Polyvinylalkohol, Poly-N-vinylpyrrolidon, Poly(N-vinylpyrrolidon-co-acrylsäure), Polyvinylinethylketon, Poly(4-vinylphenol), Poly(acrylsäure-co-styrol), Oxazolinpolymere, Polyalkylenimine, Maleinsäure- und Maleinsäureanhydridcopolymere, Hydroxyethylcellulose, Polyacetate, ionische Oberflächen- und grenzflächenaktive Verbindungen, Gallussäure oder deren Salze, Ester oder Amide, Carbonsäureester mehrwertiger Alkohole und Glycoside.

Bezüglich der erfindungsgemäß aufarbeitbaren bzw. herstellbaren Polyetherolen, d. h. Polyethermonoolen und Polyetherpolyolen sowie deren Gemischen, bestehen keinerlei Beschränkungen. Vorzugsweise eignen sich Polyetherole der allgemeinen Formel (II):

R[-O-(AO)ₙ-H]ₘ (II),

wobei R ein Alkyl-, Aryl-, Aralkyl- oder ein Alkylarylrest mit 1 bis 60, vorzugsweise 1 bis 24 Kohlenstoffatomen, AO ein oder mehrere, vorzugsweise 1 bis 3 C₂-C₁₀-Alkylenoxide und n eine ganze Zahl größer oder gleich 1, vorzugsweise 1 bis 1000, bedeutet, und m 1 bis 8 ist. Für Polyethermonoole beträgt n vorzugsweise 1 bis 100, weiter bevorzugt 1 bis 50, und m 1. Für Polyetherpolyole beträgt n vorzugsweise 1 bis 1000, weiter bevorzugt 1 bis 500, und m 2 bis 8, insbesondere 2 bis 3.

Als Verbindungen, von denen sich der Rest R ableitet, sind Alkohole und Polyalkohole, die 1 bis 60 Kohlenstoffatome aufweisen, wobei die Alkohole weitere, gegenüber Alkylenoxiden nicht reaktive funktionelle Gruppen, wie z.B. Estergruppen, aufweisen können, bevorzugt. Weiter bevorzugt werden Alkohole mit 6 bis 24 C-Atomen, besonders bevorzugt solche mit 9 bis 20 C-Atomen, eingesetzt. Derartige Alkohole können gesättigt sein, wie z.B. Methanol, Butanol, Dodecanol oder Tridecanol, Ethylenglykol, 1,2-Propylenglykol, 1,3-Propylenglykol, Butandiol, Pentandiol, Hexandiol, Glycerin, Trimethylolpropan, Pentaerythrit, Glucose,oder ungesättigt sein, wie z.B. Butenol, Vinylalkohol, Allylalkohol, Diterpenalkohole wie Geraniol, Linalool oder Citronellol. Ferner können aromatische Alkohole eingesetzt werden, vorzugsweise solche, die mit C₄-C₁₅-Alkylgruppen substituiert sind, wie z.B. Phenol oder Nonylphenol. Weitere Beispiele für einsetzbare Alkohole sind Hexanol, Heptanol, Octanol, Decanol, Undecanol, Tetradecanol, Pentadecanol, Hexadecanol, Heptadecanol, Octadecanol, Nonadecanol, Hexenol, Heptenol, Octenol, Nonenol, Decenol und Undecenol.

Hydroxyalkylester von gesättigten und ungesättigten Säuren, wie z.B. (Meth)acrylsäure, wie z.B. Hydroxyethyl(meth)acrylat, sind gleichfalls einsetzbar.

Darüber hinaus können auch Gemische derartiger Alkohole, insbesondere C₁₂-C₁₅-aliphatische Alkohole, eingesetzt werden.

Bevorzugte Polystarter sind: Wasser, Ethylenglykol, 1,2-Propylenglykol, 1,3-Propylenglykol, Butandiol, Pentandiol, Hexandiol, Glycerin, Trimethylolpropan, Pentaerythrit sowie Glucose.

Auch bezüglich der eingesetzten Alkylenoxide existieren keinerlei Beschränkungen, solange diese die oben genannten Bedingungen erfüllen. Vorzugsweise werden Ethylenoxid, Propylenoxid, Butylenoxid, Pentenoxid, Hexenoxid, Cyclohexenoxid oder deren Gemische eingesetzt. Insbesondere bevorzugt ist die Verwendung von Propylenoxid.

Vorzugsweise ist die kinematische Viskosität (Ubbelohde, 40°C) der hier in Rede stehenden Polyethermonoole kleiner als 3000 mm²/s, vorzugsweise kleiner 2000 mm²/s und insbesondere kleiner als 1000 mm²/s.

Vorzugsweise ist die dynamische Viskosität der hier in Rede stehenden Polyetherpolyole kleiner als 3000 mPa·s, vorzugsweise kleiner 2000 mPa·s und insbesondere kleiner als 1000 mPa·s.

Die Gehalte an heterogenem Katalysator im Polyetherol vor deren Abtrennung sind in Abhängigkeit von der verwendeten Katalysatorklasse unterschiedlich. Bei Verwendung von heterogenen basischen Katalysatoren beträgt deren Gehalt im als Produkt erhaltenen Polyetherol 0,05 bis 3 Gew.-%. Bei Verwendung von DMC-Katalysatoren liegt dieser Gehalt zwischen 10 und 200 ppm.

Im Rahmen des erfindungsgemäßen Verfahrens wird der heterogene Katalysator durch ein Membrantrennverfahren von Polyetherolen abgetrennt. Dabei werden als Membrantrennverfahren vorzugsweise die Mikrofiltration bzw Querstromfiltration oder Ultrafiltration eingesetzt.

Im Rahmen des erfindungsgemäß eingesetzten Membrantrennverfahrens wird vorzugsweise dem Polyetherol ein Teil desselben als Permeat entzogen und der Feststoff im verbleibenden Polyetherol (Retentat) aufkonzentriert. Das erhaltene permeat ist im wesentlichen frei vom heterogenen Katalysator. Vorzugsweise enthält dieses Permeat weniger als 20 ppm an Metall, besonders bevorzugt weniger als 10 ppm an Metall, insbesondere bevorzugt weniger als 5 ppm an Metall, jeweils bezogen auf die Gesamtmasse an Permeat.

Die Gehalte an heterogenem Katalysator im Retentat liegen zwischen 0,5 und 10 Gew.-%, bevorzugt 2 und 8 Gew.-%. Der Faktor der Aufkonzentration beträgt bei einer Katalysatorkonzentration von 0,05 % im Polyetherol nach Synthese und bei einer Konzentration von 0,5 % im Retentat 10 und bei einer Konzentration von 10 Gew.-% im Retentat 200.

Der Gehalt an Multimetallcyanidverbindung im Retentat liegt zwischen 0,5 und 10 Gew.-%, bevorzugt zwischen 2 und 8 Gew.-%. Der Faktor der Aufkonzentration beträgt bei einer Katalysatorkonzentration von 100 ppm im Polyether ex Synthese und bei einer Konzentration von 0,5 % im Retentat 50, bei einer Konzentration von 10 Gew.-% im Retentat 1000.

Der optimale Feststoffgehalt im Retentat ergibt sich je nach Polyetherol und verwendetem heterogenen Katalysator aus verschiedenen Randbedingungen. Eine dieser Randbedingungen ist beispielsweise, dass das Retentat in der Regel pumpbar bleiben sollte. Ferner können sehr hohe Feststoffgehalte im Retentat die Permeatleistung des Membranverfahrens negativ beeinflussen.

Zur Abtrennung des heterogenen Katalysators wird das das Polyetherol enthaltende Synthesegemisch unter Druck mit einer Membran in Kontakt gebracht und Permeat auf der Rückseite der Membran drucklos abgezogen. Man erhält ein Katalysatorkonzentrat (Retentat) und ein praktisch katalysatorfreies Permeat. Um einen nennenswerten Deckschichtaufbau aus Katalysator auf der Membranoberfläche zu vermeiden, der zu einer deutlichen Abnahme des Permeatflusses führt, wird durch Umpumpen, mechanische Bewegung der Membran oder Rühraggregate zwischen den Membranen eine Relativgeschwindigkeit zwischen Membran und Suspension zwischen 0,5 - 10m/s erzeugt. Die Aufkonzentration kann in Batchfahrweise durch mehrmaligen Durchgang des Synthesegemisches durch die Membranmodule oder kontinuierlich durch einmaligen Durchgang durch eine oder mehrere nacheinandergeschaltete Feed- und Bleedstufen erfolgen. Die so in Polyetherol aufkonzentrierte Katalysatorsuspension kann anschließend wieder zur Synthese von Polyetherol eingesetzt werden.

Für das Membranverfahren der Mikrofiltration bzw. Querstromfiltration werden Membrantrennschichten mit Porendurchmesser zwischen 5000 nm und 100 nm, für das Membranverfahren der Ultrafiltration Membrantrennschichten der Porendurchmesser zwischen 100 nm und 5 nm eingesetzt. Die Membranen können in Flach-, Rohr-, Multikanal-, Kapillar- oder Wickelgeometrie eingesetzt werden, für die entsprechende Druckgehäuse, die eine Trennung zwischen Katalysatorsuspensionen und dem Permeat (Filtrat) erlauben, verfügbar sind. Die aufzubringenden transmembranen Drücke zwischen Retentat und Permeat liegen im wesentlichen abhängig von Durchmesser der Membranporen und der mechanischen Stabilität der Membran bei der Betriebstemperatur je nach Membranart zwischen 0,5 und 60 bar. Die Betriebstemperatur ist abhängig von der Produkt- und der Membranstabilität. Dabei erhöhen sich die Permeatflüsse drastisch mit Zunahme der Temperatur. Mit Keramikmembranen sind z.B. die Temperaturen bis 140 °C realisierbar.

Die Trennschichten können aus organischen Polymeren, Keramik, Metall oder Kohlenstoff bestehen. Aus mechanischen Gründen sind die Trennschichten in der Regel auf einer ein- oder mehrschichtigen Unterstruktur aus dem gleichen oder einem oder mehreren unterschiedlichen Materialien verglichen mit dem Material der Trennschicht aufgebracht. Beispiele sind:

| | |
|---|---|
| Trennschicht | Unterstruktur (gröber als Trennschicht) |
| Metall | Metall |
| Keramik | Metall, Keramik oder Kohlenstoff |
| Polymer | Polymer, Metall, Keramik oder Keramik auf Metall |
| Kohlenstoff | Kohlenstoff, Metall oder Keramik |
| Keramik: z.B. α-Al₂O₃, γ-Al₂O₃, ZrO₂, TiO₂, SiC, gemischte keramische Werkstoffe Polymer: z.B. PTFE, PVDF, Polysulfon | |

Da hohe Temperaturen vorteilhaft sind wegen der damit verbundenen höheren Permeatflüssen ist der Einsatz rein anorganischer Membranen ( einschließlich Kohlenstoff) bevorzugt.

Das erfindungsgemäße Verfahren zur Herstellung von Polyetherolen, umfassend Polyethermonoole und Polyetherpolyole, umfasst als einen Schritt auch das erfindungsgemäße Aufarbeitungsverfahren. Die eigentliche Herstellung der Polyetherole erfolgt nach den im Stand der Technik bekannten kontinuierlichen oder diskontinuierlichen Verfahren, wobei die kontinuierliche Fahrweise bevorzugt ist. Diesbezüglich verweisen wir auf den eingangs zitierten Stand der Technik gemäß der DE-A 199 57 105.8, der DE-A 198 40 846.3 und die WO 98/44022 sowie die darin zitierten Druckschriften.

Insbesondere betrifft die vorliegende Erfindung ein Verfahren zur Herstellung von Polyetherolen, in das der erfindungsgemäß aufgearbeitete Katalysator entweder als solcher oder in Form einer Suspension in einem oder mehreren Polyetherolen als Katalysator in einer nachfolgenden Herstellung von Polyetherolen verwendet wird, also in das Herstellungsverfahren nach Aufarbeitung zurückgeführt wird. Ein derartiges Verfahren zeichnet sich durch eine besonders hohe Wirtschaftlichkeit aus.

Die hier in Rede stehenden Polyetherole finden beispielsweise Verwendung als Trägeröle in Kraftstoffadditivmischungen, wobei es überaus vorteilhaft ist, den verwendeten Katalysator aus dem als Reaktionsprodukt erhaltenen Polyetherol zu entfernen. Dies gilt im besonderen Maße für die als Trägeröle verwendeten Polyetherole, da Katalysatorrückstände einerseits korrosiv wirken können und andererseits bei Verbrennung im Motorraum zu unerwünschten Ablagerungen und Emissionen führen können. Insbesondere finden sie auch Verwendung in der Automobilindustrie als Treibstoffadditiv, in Bremsflüssigkeiten, in Polyurethanen, insbesondere Weichschaumstoffen, als Tenside oder Lösemittel.

Die Erfindung soll nunmehr anhand der folgenden Beispiele näher erläutert werden.

### Beispiele

### Herstellbeispiel 1: Herstellung der Hexacyanocobaltatsäure

7 1 stark saurer Ionenaustauscher, der sich in der Natriumform befand, (Amberlite 252 Na, Fa. Rohm&Haas) wurden in eine Austauschersäule (Länge 1 m, Volumen 7,7 1) gefüllt. Der Ionenaustauscher wurde anschließend in die H-Form überführt, indem 10 %ige Salzsäure mit einer Geschwindigkeit von 2 Bettvolumen pro Stunde 9 h lang über die Austauschersäule gefahren wurde, bis der Na-Gehalt im Austrag kleiner 1 ppm betrug. Anschließend wurde der lonenaustauscher mit Wasser neutral gewaschen.

Der regenerierte Ionenaustauscher wurde nun benutzt, um eine im wesentlichen alkalifreie Hexacyanocobaltatsäure herzustellen. Dazu wurde eine 0,24 molare Lösung von Kaliumhexacyanocobaltat in Wasser mit einer Geschwindigkeit von einem Bettvolumen pro Stunde über den Austauscher gefahren. Nach 2,5 Bettvolumen wurde von der Kaliumhexacyanocobaltat-Lösung auf Wasser gewechselt. Die erhaltenen 2,5 Bettvolumen hatten im Mittel einen Gehalt von Hexacyanocobaltatsäure von 4,5 Gew.-% und Alkaligehalte kleiner 1 ppm. Die für die weiteren Beispiele verwendeten Hexacyanocobaltatsäure-Lösungen wurden entsprechend mit Wasser verdünnt.

### Herstellbeispiel 2: Herstellung einer Suspension der Multimetallcyanidverbindung aus Herstellbeispiel 1 in Tridekanol N

In einem Rührkessel mit einem Volumen von 30 1, ausgestattet mit einem Scheibenrührer, Tauchrohr für die Dosierung, pH-Sonde und Streulicht-Sonde, wurden 1600 g wässrige Hexacyanocobaltsäure (Cobalt-Gehalt: 9 g/I Cobalt) vorgelegt und unter Rühren auf 50°C erwärmt. Anschließend wurden unter Rühren (Rührleistung 1 W/I) 9224 g wässrige Zinkacetat-Dihydrat-Lösung (Zinkgehalt: 2,6 Gew.-%), welche ebenfalls auf 50 °C temperiert war, innerhalb von 15 min zugefahren.

Anschließend wurden 351 g Pluronic PE 6200 (BASF AG) unter Rühren zugegeben. Dann wurden 3690 g wässrige Zinkacetat-Dihydrat-Lösung (Zinkgehalt: 2,6 Gew.-%) wurden dann unter Rühren (Rührenergie: 1 W/I) bei 50 °C innerhalb 5 min zudosiert. Die Suspension wurde bei 50°C solange nachgerührt bis der pH-Wert von 4,0 auf 3,2 gefallen war und konstant blieb. Die so erhaltene Fällsuspension wurde anschließend abfiltriert und auf dem Filter mit dem 6-fachen Kuchenvolumen an Wasser gewaschen.

Der wasserfeuchte Filterkuchen wurde anschließend in einer Rührapparatur mit 30 I Volumen in 20 kg Tridekanol N (BASF AG) dispergiert und bei einer Temperatur von 80°C im Vakuum entwässert.

Die so erhaltene Suspension der Multimetallcyanidverbindung in Tridekanol N wurde anschließend mittels Spalt-Rotor-Mühle erneut dispergiert. Die dabei erhaltene Suspension hatte einen Multimetallcyanidgehalt von 5 Gew.-%.

### Herstellbeispiel 3: Herstellung eines als Trägeröl verwendeten Polyethermonools mittels Multimetallcyanid-Katalysator aus Herstellbeispiel 2

In einen Rührreaktor, ausgestattet mit einem außenliegenden Wärmetauscher und einem Volumen von 600 I, wurden 100,00 kg Tridekanol N eingesaugt und 2,14 kg einer 5%igen Multimetallcyanid-Suspension in Tridekanol N, dies entspricht 200 ppm Katalysator bezogen auf die Endmenge, zugegeben. Anschließend wurde unter vermindertem Druck (< 50 mbar) bei 115° C bis zu einem Wassergehalt < 0,02% entwässert. Das Vakuum wurde unter Stickstoff Zufuhr aufgehoben.

Nach anschließender Druckprüfung wurde bei einem Anfangsdruck von 0,5 bar auf 135° C aufgeheizt. Zur Reaktionskontrolle (Anspringen der Reaktion) wurden ca. 24 kg Propylenoxid bei 135° C zugefahren, das nach einer Anlaufzeit von ca. 6 Minuten unter Druckabfall abreagierte. Die Differenz der benötigten Propylenoxid-Menge von 411,3 kg (Gesamtmenge: 435,2 kg PO) wurde in Folge über PO-Dosier-Rampen bei 135° C innerhalb von ca. 3 Stunden bei einem max. Druck von ca. 2 bar zugefahren. Danach wurde auf 80° C abgekühlt, unter Stickstoff entspannt und ca. 60 Minuten unter vermindertem Druck (< 30 mbar) gestrippt.

Die Aufarbeitung des so erhaltenen Trägeröls mittels Querstrom-Filtration wird im folgenden Beispiel 1 ausführlich beschrieben.

### Beispiel 1: Abtrennung des Multimetallcyanid-Katalysators aus Herstellbeispiel 3 mittels Membranfiltration

Der Austrag des 200 ppm Katalysator enthaltenen Synthesesuspension wurde in Batchfahrweise auf ca. 3,5 % aufkonzentriert. Eingesetzt wurde eine keramische Membran mit einer Trenngrenze (Porendurchmesser) von 50 nm. Die Trennschicht bestand aus ZrO₂, die auf der Innenseite runder Kanäle mit 6 mm Durchmesser auf einer mehrschichtigen Unterstruktur aus alpha-Al₂O₃ aufgebracht worden war. Durch die Membrankanäle wurde die Suspension mit 2 m/s über ein Kreislaufgefäß gepumpt. Die Prozesstemperatur betrug 70 °C, der transmembrane Druck 3 bar. Der im Permeat ermittelte Co- bzw. Zn-Gehalt war zum Anfang und zum Ende der Aufkonzentration < 1 ppm.

### Angaben zu physikalisch-chemischen Kennzahlen (nach Membranfiltration):

| | |
|---|---|
| Kinematische Viskosität bei 40° C | 56,5 mm²/s |
| Restmetallgehalt | < 1 ppm |
| Hydroxylzahl | 52 mg KOH/g |
| Jodzahl n. Kaufmann (Doppelbindungsanteile) | 0,1 g Jod/100 g |
| Gehalt an Propylenoxid | 1,8 ppm |
| Wassergehalt | 0,02 |
| Dichte bei 20° C | 0,9658 g/cm³ |

### Beispiel 2: Wiedereinsatz des querstromfiltrierten Katalysators aus Beispiel 1

In einem Rührreaktor mit einem Volumen von ca. 2 1 wurden 200 g Tridekanol N und 6,11 g der oben beschriebenen aus der Querstromfiltration erhaltenen Multimetallcyanid-Katalysator-Suspension (ca. 3,5 %ig) vorgelegt Dies entsprach 200 ppm Multimetallcyanid bezogen auf das Endmengengerüst. Anschließend wurde auf 120° C erhitzt und bei dieser Temperatur 2 Stunden bis zu einem Wassergehalt < 0,05% entwässert. Das Vakuum wurde unter Stickstoff Zufuhr aufgehoben. Nach anschließender Druckprüfung wurde bei einem Anfangsdruck von 0,5 bar auf 135° C aufgeheizt. Zur Rektionskontrolle (Anspringen der Reaktion) wurden ca. 50 g Propylenoxid bei 135° C zugefahren, das nach einer Anlaufzeit von ca. 6 Minuten unter Druckabfall abreagierte. Die Differenz der benötigten Propylenoxid-Menge von 820 g (Gesamtmenge: 870 g PO) wurde in Folge über PO-Dosier-Rampen bei 135° C innerhalb von ca. 3 Stunden bei einem max. Druck von ca. 8 bar zugefahren. Danach wurde 2 Stunden bei 135° C nachgerührt, unter Stickstoff entspannt und das Reaktionsgemisch im Labor bei ca. 100° C und unter vermindertem Druck < 30 mbar in einem Rotationsverdampfer gestrippt, und abschliessend tiefenfiltriert.

### Angaben zu physikalisch-chemischen Kennzahlen:

| | |
|---|---|
| Kinematische Viskosität bei 40° C | 60 mm²/s |
| Restmetallgehalt | Co < 2 ppm, Zn 4 ppm |
| Hydroxylzahl | 51 mg KOH/g |
| Jodzahl n. Kaufmann (Doppelbindungsanteile) | < 0,1 g Jod/100 g |
| Gehalt an Propylenoxid | < 1 ppm |
| Wassergehalt | 0,07% |
| Dichte bei 20° C | 0,9677 g/cm³. |

### Herstellbeispiel 4: Herstellung einer Suspension der Multimetallcyanidverbindung aus Herstellbeispiel 1 in einem Glycerinpropoxylat-Polymer

In einem Rührkessel mit einem Volumen 800 1, ausgestattet, mit einer Schrägblattturbine, Tauchrohr für die Dosierung, pH-Elektrode, Leitfähigkeitrneßzelle und Streulicht-Sonde, wurden 370 kg wässrige Hexacyanocobaltsäure (Cobaltgehalt: 9 g/l Cobalt) vorgelegt und unter Rühren auf 50°C erwärmt. Anschließend wurden unter Rühren (Rührleistung 1 W/l) 209,5 kg wässrige Zinkacetat-Dihydrat-Lösung (Zinkgehalt: 2,7 Gew.-%) welche auf ebenfalls 50 °C temperiert war, innerhalb von 50 min zugefahren.

Anschließend wurden 8 kg Pluronic PE 6200 (BASF AG) und 10,7 kg Wasser unter Rühren zugegeben. Dann wurden 67,5 kg wässrige Zinkacetat-Dihydrat-Lösung (Zinkgehalt; 2,7, Gew.-%) unter Rühren (Rührenergie 1W/1) bei 50 °C innerhalb 20 min zudosiert.

Die Suspension wurde bei 50 °C solange nachgerührt bis der pH-Wert von 3,7 auf 2,7 gefallen war und konstant blieb. Die so erhaltene Fällsuspension wurde anschließend mittels einer Filterpresse abfiltriert und in der Filterpresse mit 400 l Wasser gewaschen.

Der wasserfeuchte Filterkuchen wurde mittels Spalt-Rotor-Mühle in einer aktive Wasserstoffatome aufweisenden Starterverbindung (Glycerinpropoxylat-Polymer mit MG: 900g/mol) dispergiert. Die dabei erhaltene Suspension hatte einen Multimetallcyanidgehalt von ca. 5 Gew.-%.

### Herstellbeispiel 5: Herstellung eines Polyetherpolyols mittels Multimetallcyanid-Katalysator aus Herstellbeispiel 4

Die Synthese wurde in einem gereinigten und getrockneten 20-1-Rührautoklaven durchgeführt. Es wurden 2,0 kg eines propoxylierten Glycerins mit einem Molekulargewicht M_{w} von 400 g/Mol sowie 0,196 kg propoxyliertes Ethylenglykol der Molmasse 250 g/mol in den Rührkessel gegeben und mit 38 g Multimetallcyanidkatalysator-Suspension aus Herstellbeispiel 4 versetzt. Der Kesselinhalt wurde mit Stickstoff inertisiert und insgesamt 1,5 h lang bei 110 °C im Vakuum behandelt. Bei 115 °C wurden 3,5 bar Stickstoff zugegeben und anschließend innerhalb von 3,5 Stunden erst 3,45 kg Propylenoxid, anschließend 12,1 kg eines Gemischs aus 10,2 kg Propylenoxid und 1,9 kg Ethylenoxid zudosiert. Anschließend wurden 2,0 kg Propylenoxid angelagert. Es wurden weitere 0,6 h gerührt und bei 115 °C und 9 mbar entgast. Der entstandene Polyetheralkohol wies folgende Kennwerte auf:
Hydroxylzahl: 47,4 mg KOH/g
Dynamische Viskosität bei 25 °C: 578 mPas
Gehalt an Zn und Co: 27 ppm bzw. 12 ppm

Nach der Filtration erhielt man ein Polymer mit Zn und Co unterhalb der Nachweisgrenze:
Hydroxylzahl: 47,4 mg KOH/g
Dynamische Viskosität bei 25 °C: 578 mPas.

### Beispiel 3: Abtrennung des Multimetallcyanid-Katalysators mittels Membranfiltration aus Herstellbeispiel 5

Der Austrag des 100 ppm Katalysator enthaltenen Synthesesuspension wurde in Batchfahrweise auf ca. 3 % aufkonzentriert. Eingesetzt wurde eine keramische Membran mit einer Trenngrenze (Porendurchmesser) von 50 nm. Die Trennschicht bestand aus ZrO₂, die auf der Innenseite runder Kanäle mit 6 mm Durchmesser auf einer mehrschichtigen Unterstruktur aus alpha-Al₂O₃ aufgebracht worden war. Durch die Membrankanäle wurde die Suspension mit 2 m/s über ein Kreislaufgefäß gepumpt. Die Prozesstemperatur betrug 70 °C, der transmembrane Druck lag bei 3 bar. Der im Permeat ermittelte Co- bzw. Zn-Gehalt war zum Anfang und zum Ende der Aufkonzentration < 1 ppm.

### Beispiel 4: Wiedereinsatz des querstromfiltrierten Katalysators aus Beispiel 3

Die Synthese wurde in einem gereinigten und getrockneten 20-1-Rührautoklaven durchgeführt. Es wurden 2,0 kg eines propoxylierten Glyzerins mit einem Molekulargewicht M_{w} von 400 g/Mol sowie 0,196 kg propoxyliertes Ethylenglykol der Molmasse 250 g/mol in den Rührkessel gegeben und mit ca. 64 g der abgetrennten Katalysatorsuspension versetzt. Dies entsprach 100 ppm Multimetallcyanidkatalysator bezogen auf die Endmenge. Der Kesselinhalt wurde mit Stickstoff inertisiert und insgesamt 1,5 h bei 110 °C im Vakuum behandelt. Bei 115 °C wurden 3,5 bar Stickstoff zugegeben und anschließend innerhalb von 3,5 Stunden erst 3,45 kg Propylenoxid, anschließend 12,1 kg eines Gemischs aus 10,2 kg Propylenoxid und 1,9 kg Ethylenoxid zudosiert. Anschließend wurden 2,0 kg Propylenoxid angelagert. Es wurden weitere 0,6 h gerührt und bei 115 °C und 9 mbar entgast. Der entstandene Polyetheralkohol wies folgende Kennwerte auf:
Hydroxylzahl: 47,2 mg KOH/g
Dynamische Viskosität bei 25 °C: 605 mPas
Gehalt an Zn und Co: 27 ppm bzw. 12 ppm

Nach der Filtration erhielt man ein Polymer mit Zn und Co unterhalb der Nachweisgrenze:
Hydroxylzahl: 47,2 mg KOH/g
Dynamische Viskosität bei 25 °C: 605 mPas.

Der in den Beispielen 1 bis 4 angegebene Metallgehalt wurde mit Hilfe der Atomemissionsspektroskopie und der Methode des induktiv gekoppelten Plasmas (ICP) bestimmt.

Die in den Beispielen 1 und 2 angegebene kinematische Viskosität wurde mit Hilfe eines Viskosimeters nach Ubbelohde analog DIN 51562 gemessen.

Die in den Beispielen 3 und 4 angegebene dynamische Viskosität wurde mit Hilfe eines Rotationsviskometers mit Kegel-Platte-Meßeinrichtung (Rheolab-MC-1 der Firma Physica) analog DIN 53018 und 53019 gemessen.

## Patentansprüche

1. Verfahren zur Aufarbeitung von Polyetheralkoholen (Polyetherole), die durch ring-öffnende Polymerisation von Epoxiden mit OH-funktionellen Startern unter Verwendung mindestens eines heterogenen Katalysators hergestellt wurden, **dadurch gekennzeichnet, dass** der mindestens eine heterogene Katalysator durch ein Membrantrennverfahren vom Polyetherol abgetrennt wird, wobei der heterogene Katalysator ein Multimetallcyanid-Katalysator ist.

2. Verfahren nach Anspruch 1, wobei der heterogene Katalysator nach der ring-öffnenden Polymerisation chemisch nicht verändert wird.

3. Verfahren nach Anspruch 1 oder 2, wobei das Membrantrennverfahren ausgewählt wird aus der Gruppe bestehend aus Mikrofiltration bzw. Querstromfiltration und Ultrafiltration.

4. Verfahren nach einem der Ansprüche 1 bis 3, wobei die im Membrantrennverfahren eingesetzten Membranen Trennschichten aufweisen, deren Porendurchmesser 5 bis 5000 nm beträgt.

5. Verfahren nach einem der Ansprüche 1 bis 4, wobei die Trennschichten der im Membrantrennverfahren eingesetzten Membranen aus organischen Polymeren, einem keramischen Material, Metall oder Kohlenstoff oder einer Kombination aus zwei oder mehr davon bestehen.

6. Verfahren nach einem der Ansprüche 1 bis 5, wobei die Abtrennung des heterogenen Katalysators vom Polyetherol kontinuierlich oder diskontinuierlich erfolgt.

## Claims

1. A process for working up polyether alcohols (polyetherols) which have been prepared by ring-opening polymerization of epoxides by means of OH-functional starters using at least one heterogeneous catalyst, wherein the at least one heterogeneous catalyst is separated off from the polyetherol by means of a membrane separation process, the heterogeneous catalyst being a multimetal cyanide catalyst.

2. A process as claimed in claim 1, wherein the heterogeneous catalyst is not changed chemically after the ring-opening polymerization.

3. A process as claimed in claim 1 or 2, wherein the membrane separation process is selected from the group consisting of microfiltration, crossflow filtration and ultrafiltration.

4. A process as claimed in any of claims 1 to 3, wherein the membranes used in the membrane separation process have separation layers whose pore diameter is from 5 to 5000 nm.

5. A process as claimed in any of claims 1 to 4, wherein the separation layers of the membranes used in the membrane separation process comprise organic polymers, a ceramic material, metal or carbon or a combination of two or more thereof.

6. A process as claimed in any of claims 1 to 5, wherein the separation of the heterogeneous catalyst from the polyetherol is carried out continuously or batchwise.

## Revendications

1. Procédé de traitement d'alcools de polyéther (polyéthérols), préparés par polymérisation à ouverture de cycle d'époxydes avec des amorces à fonctionnalité OH en utilisant au moins un catalyseur hétérogène, **caractérisé en ce que** le au moins un catalyseur hétérogène est séparé du polyéthérol par un procédé de séparation à membrane, le catalyseur hétérogène étant un catalyseur de cyanure multimétallique.

2. Procédé selon la revendication 1, **caractérisé en ce que** le catalyseur hétérogène n'est pas chimiquement modifié après la polymérisation à ouverture de cycle.

3. Procédé selon la revendication 1 ou 2, dans lequel le procédé de séparation à membrane est choisi dans le groupe formé par une microfiltration ou une filtration à courant transversal et une ultrafiltration.

4. Procédé selon l'une quelconque des revendications 1 à 3, dans lequel les membranes mise en oeuvre dans le procédé de séparation à membrane présentent des couches de séparation dont le diamètre de pores est de 5 à 5000 nm.

5. Procédé selon l'une quelconque des revendications 1 à 5, dans lequel les couches de séparation des membranes mises en oeuvre dans le procédé de séparation à membrane sont constituées de polymères organiques, d'un matériau céramique, de métal ou de carbone ou d'une combinaison de deux ou plus de deux d'entre eux.

6. Procédé selon l'une quelconque des revendications 1 à 5, dans lequel la séparation du catalyseur hétérogène et du polyéthérol est entreprise en mode continu ou discontinu.
